# EUROPÄISCHE PATENTSCHRIFT

(11) **EP 1 429 142 B1**
(45) Veröffentlichungstag und Bekanntmachung des Hinweises auf die Patenterteilung: **02.09.2009**
(21) Anmeldenummer: 02027762.0
(22) Anmeldetag: 11.12.2002
(51) Int. Cl.: G01N 33/20

(54) **Verfahren zur Messung der von einem metallischen Werkstück bei einer thermochemischen Behandlung aufgenommenen Menge einer Komponente**
Method for measuring the quantity of a component absorbed by a metallic workpiece during thermochemical treatment
Procédé de mesure d'une quantité de composant absorbé par une pièce métallique pendant un traitement thermochimique

(43) Veröffentlichungstag der Anmeldung: 16.06.2004
(73) Patentinhaber: Ipsen International GmbH, 47533 Kleve (DE)
(72) Erfinder: Feichtinger, Heinrich, Prof. Dr. Ing., 8132 Hinteregg (CH)
(74) Vertreter: Stenger, Watzke & Ring

(56) Entgegenhaltungen:
- EP-A- 0 558 130
- US-A- 5 378 889
- DATABASE WPI Section Ch, Week 200225 Derwent Publications Ltd., London, GB; Class M24,Page 1, AN 2002193767 XP002241703 & KR 2001 037 229 A (POHANG IRON & STEEL CO LTD), 7. Mai 2001 (2001-05-07)
- NETZSCH GERAETEBAU GMBH: "Dilatometer DIL 402 PC" INTERNET SEITE, [Online] 6. Oktober 2000 (2000-10-06), XP002241473 Gefunden im Internet: <URL:www.e-thermal.com/dil402pc.htm> [gefunden am 2003-05-16]

## Beschreibung

Die Erfindung betrifft ein Verfahren zur Messung der bei einer thermochemischen Behandlung metallischer Werkstücke aus der die Werkstücke umgebenden Gasatmosphäre abgegebenen und von den Werkstücken aufgenommenen Menge einer Komponente.

Thermochemische Prozesse, wie beispielsweise das Einsatzhärten, das Nitrieren oder das Carbonitrieren sind von großer Bedeutung, denn erlauben sie es, einem Bauteil im oberflächennahen Bereich spezielle mechanische, physikalische und/oder chemische Eigenschaften zu verleihen. Eine Vielzahl thermochemischer Prozesse erfolgt dabei unter Temperatureinwirkung durch Abscheidung einer Komponente oder mehreren Komponenten aus der Gasphase, infolgedessen lösliche Atome der Komponenten, z. B. Kohlenstoff oder Stickstoff in Lösung gehen und mit Anteilen der Legierungselemente der Werkstücke Verbindungen, z. B. Carbide, Nitrite oder Carbonitrite bilden.

Die Erzielung gewünschter Eigenschaften hängt in erster Linie von der Geometrie sowie der Zusammensetzung der erzeugten Oberflächenschicht, d. h. vom Konzentrationsprofil sowie der Bindungsart der Komponente im oberflächennahen Bereich ab. Das Konzentrationsprofil ist dabei das Resultat der Zusammensetzung, der Temperatur und Zeitdauer der auf das Bauteil einwirkenden Gasatmosphäre und kann durch entsprechende Wahl der Parameter Zeit, Temperatur, Druck sowie Gaszusammensetzung eingestellt werden.

Die Einstellung der vorgenannten Parameter kann jedoch in vielen Fällen nicht so exakt vorgenommen werden, daß eine hinreichende Aussage über das Resultat einer thermochemischen Behandlung getroffen werden könnte. Ein wesentlicher Grund hierfür ist, daß thermochemische Prozesse im starken Ungleichgewicht ablaufen und daß ihre Kinetik unter anderem auch vom Zustand der Oberfläche des zu behandelnden Werkstückes abhängt. Hinzu kommen Konvektionsverhältnisse im Reaktor sowie das Vorhandensein von fremden reaktiven Oberflächen, an welchen das Behandlungsgas in unkontrollierter Weise zur Reaktion kommt.

Um die vorgenannten Störgrößen zumindest zum Teil rechnerisch eliminieren zu können, sind insbesondere beim Einsatzhärten unterschiedliche Meß- und Auswertungsmethoden entwickelt worden, die eine exakte Vorhersage des Verfahrensablaufes in nachteiliger Weise jedoch nicht ermöglichen.

Beim beispielsweise Einsatzhärten werden die zu behandelnden Werkstücke bei Temperaturen im Bereich von 900°C bis 1100°C einer kohlenstoffhaltigen Atmosphäre ausgesetzt. Als Kohlenstofflieferant können dabei Kohlenwasserstoffe, wie z. B. Methan oder Propan dienen, welche bei der Verfahrenstemperatur unter Bildung von Wasserstoff Kohlenstoffatome an die Metalloberfläche abgeben. Infolge physikalischer und chemischer Absorption werden die Kohlenstoffatome abgespalten und dringen anschließend über einen Diffusionsvorgang in das Metall ein, wobei ein Konzentrationsprofil entsteht, welches nach der Abkühlung ein entsprechendes Härteprofil bildet. Sind neben dem Kohlenwasserstoff noch Sauerstoffträger, z. B. Kohlensäure und/oder Wasserdampf vorhanden, so erfolgt der eigentliche Aufkohlungsvorgang durch Kohlenmonoxid, welches durch Reaktion mit dem Sauerstoffträger gebildet wird. Die eigentliche Aufkohlungsreaktion erfolgt dann durch die Spaltung des absorbierten Kohlenmonoxid-Moleküls an der Oberfläche des Werkstücks, wobei ein Sauerstoffatom freigesetzt wird. Durch die Entfernung dieses Sauerstoffs von der Oberfläche wird wesentlich die Geschwindigkeit des Abspaltungsvorgangs und damit die Aufkohlung der Randschicht bestimmt. Dies kann entweder durch Reaktion des absorbierten Kohlenmonoxids-Moleküls mit einem weiteren Kohlenmonoxid-Molekül zu Kohlensäure oder durch Reaktion mit einem Wasserstoffmolekül zu Wasserdampf geschehen, wobei letztere Reaktion sehr schnell abläuft. Das neu gebildete Wasser kann mit frisch zugeführtem Kohlenwasserstoff wieder zu Kohlenmonoxid und Wasserstoff regeneriert werden, womit der Reaktionskreislauf geschlossen ist.

Bei Kenntnis der Legierungszusammensetzung sowie der Aufkohlungstemperatur kann über die Messung der Partialdrücke der Kohlenstoffgehalt berechnet werden, welcher sich im Gleichgewicht einstellt. Dieser Kohlenstoffgehalt wird als C-Pegel bezeichnet. Im Bereich der Kohlenstoffaktivität des Aufkohlungsvorganges ist das Wasser fast komplett dissoziert, d. h. die Mengenanteile von Kohlenmonoxid und Wasserstoff können als konstant angenommen werden. Dadurch können die geringen Restwasserstoffgehalte als für die Kohlenstoffaktivität bestimmend angenommen werden, wobei die Wasserstoffgehalte z. B. mit der Taupunkt-Meßmethode genau bestimmt werden können.

Im analogen Aufkohlungssystem Kohlenmonoxid/Kohlensäure, welches dem Boudouard-Gleichgewicht entspricht, liegt Kohlensäure im Gegensatz zu den anderen Gasen in nur geringer Menge vor, welche für die Kohlenstoffaktivität bestimmend ist. Die Bestimmung der geringen Kohlensäuremenge kann durch Infrarot-Gasanalyse erfolgen.

Eine weitere Methode der Bestimmung der Kohlenstoffaktivität ergibt sich durch die Nutzung der Gleichgewichte mit Sauerstoff, denn ergibt sich beispielsweise für das die Kohlenstoffaktivität bestimmende Verhältnis von Wasserstoff und Wasserdampf, das dieses bei einer bestimmten Temperatur im Gleichgewicht mit dem Sauerstoffpartialdruck steht. Der Sauerstoffpartialdruck kann dabei durch Messung mit einer Feststoffelektrolyt-Sonde erfaßt werden.

All die vorbeschriebenen Meßverfahren, die die aufkohlende Wirkung der Atmosphäre zu erfassen versuchen, setzen voraus, daß die Atmosphäre im Gleichgewicht mit der Oberfläche des zu behandelnden Werkstückes steht. Die effektive Kohlenstoffkonzentration, die sich im Zuge der Verfahrensdurchführung an der Metalloberfläche tatsächlich einstellt, hängt jedoch einerseits von der Geschwindigkeit des Kohlenstoff-Antransport aus der Atmosphäre und andererseits von der Geschwindigkeit, mit der dieser Kohlenstoff durch Diffusion in das Metallinnere eindifundiert, ab. Dabei ist entscheidend, wie schnell der beim Lösungsvorgang des Kohlenstoffs frei werdende Sauerstoff von der Oberfläche entfernt wird. Zudem ist für die tatsächlich erzielte Aufkohlung von Bedeutung, ob die Entfernung des Sauerstoffes von der Oberfläche durch Reaktion mit Kohlenmonoxid oder mit Wasserstoff erfolgt. Zudem spielen die Konvektionverhältnisse im Reaktor eine gleichfalls einflußnehmende Rolle.

Die Messung der Veränderung der Gaszusammensetzung im Reaktor ist mithin nur eine indirekte und in vielen Fällen in nachteiliger Weise auch nur eine ungenaue Beschreibung der sich tatsächlich an der Werkstoffoberfläche abspielenden Vorgänge. Dabei können sich die Vorgänge als noch komplexer einstellen, wenn es im Wärmebehandlungsofen durch überhöhte Kohlenstoffaktivität zur Abscheidung von Ruß kommt oder wenn ein Teil des Aufkohlungsgases dazu benutzt wird, Oberflächenoxide des Werkstückes zu reduzieren.

Die aus dem Stand der Technik vorbekannten Meß- und Auswerteverfahren sind somit nicht dazu geeignet, eine hinreichende Vorhersage über den Ablauf eines thermochemischen Prozesses treffen zu können.

Es ist aus der EP-A-0 558 130 ferner eine Vorrichtung bekannt geworden, die der Bestimmung der Reaktivität von Luft, der Reaktivität von Co₂, des Rußindexes und der Koeffizienten der thermischen Expansion eines Kohlenstoffproduktes dient. Die vorstehenden Probleme lassen sich aber auch mit dieser vorbekannten Vorrichtung nicht beseitigen.

Hiervon ausgehend ist es daher **Aufgabe** der Erfindung, ein Meßverfahren anzugeben, das auch im laufenden Behandlungsprozeß die Möglichkeit bietet, eine weitestgehend exakte Angabe über die Menge einer aus der Gasatmosphäre abgegebenen und von den Werkstücken aufgenommenen Komponente geben zu können.

Zur **Lösung** dieser Aufgabe wird mit der Erfindung vorgeschlagen ein Verfahren zur kontinuierlichen Messung der bei einer thermochemischen Behandlung metallischer Werkstücke aus der die Werkstücke umgebenden Gasatmosphäre abgegebenen und von den Werkstücken aufgenommenen Menge einer Komponente, bei dem ein Probenkörper, dessen Längserstreckung seine Quererstreckung wesentlich übersteigt, der Wirkung der Gasatmosphäre ausgesetzt, die infolge der Aufnahme der aus der Gasatmosphäre abgegebenen Komponente verursachte zeitliche Längenänderung des Probenkörpers in Längsrichtung gemessen und die gemessene Längenänderung zur Bestimmung der Menge der aus der Gasatmosphäre in den Probenkörper übertragenen Komponente verwendet wird.

Das erfindungsgemäße Verfahren basiert auf der technischen Lehre, daß der Probenkörper infolge der Aufnahme einer aus der Gasatmosphäre stammenden Komponente einer Volumensänderung unterliegt, welche sich in Längsrichtung des Probekörpers als Längenänderung manifestiert, die direkt erfaßt und zur Bestimmung der Menge an übertragener Komponente verwendet werden kann. Die Volumensänderung entsteht dabei durch Aufweitung des Eisengitters bei der Einlagerung der Komponentenatome und/oder durch die Entstehung einer Ausscheidung, welche die Komponente mit einem oder mehreren Legierungselementen der Probe bildet. Das erfindungsgemäß vorgeschlagene Verfahren ermöglicht mithin, die aus der Gasatmosphäre übergegangene Menge einer Komponente direkt zu messen, was gegenüber den aus dem Stand der Technik bekannten Meß- und Auswertemethoden den Vorteil hat, daß die durch Druck, Temperatur, reaktive Oberflächen und dergleichen bestimmte Störgrößen keinerlei Einfluß auf das Meßergebnis haben. Das erfindungsgemäße Verfahren ermöglicht daher eine exakte Bestimmung der auf den Probenkörper und damit auch auf die Werkstücke übergegangene Menge der aus der Gasatmosphäre stammenden Komponente. Die Mengenbestimmung kann dabei in vorteilhafter Weise kontinuierlich durchgeführt werden, was die Möglichkeit einer weitestgehend exakten Vorhersage eröffnet, denn können anhand zeitlich erfaßter Verläufe Prognosen berechnet werden, die infolge der hohen Meßgenauigkeit des erfindungsgemäßen Verfahrens eine nur geringe Fehlerträchtigkeit aufweisen.

Zur Bestimmung der Längenänderung eines Probenkörpers wird dieser in einer dafür ausgebildeten Aufnahme eingespannt. Die Aufnahme besteht aus zwei Widerlagern, wobei eines der Widerlager positionsgenau fixiert ist und das andere Widerlager gegenüber dem fixierten Widerlager relativ bewegbar angeordnet ist. Infolge einer Längenausdehnung des zwischen den beiden Widerlagern angeordneten Probenkörpers wird sich das eine Widerlager gegenüber dem anderen Widerlager verschieben. Der sich insgesamt infolge einer Längenänderung des Probenkörpers ergebende Verschiebeversatz der beiden Widerlager zueinander entspricht der insgesamt aufgetretenen Längenänderung des Probenkörpers. Zustande kommt die Längenänderung des Probenkörpers durch Aufnahme einer aus der Gastmosphäre stammenden Komponente, wobei die Komponente senkrecht zur Längsachse des Probenkörpers in diesen eindiffundiert. Einhergehend mit dieser Eindiffusion der Komponente ist eine Volumensänderung des Probenkörpers, die auch in den beiden senkrecht zur Längsachse des Probenkörpers ausgebildeten Stirnflächen eine Änderung des Oberflächenprofils bewirkt. Zu Beginn sind die beiden Stirnflächen des Probenkörpers eben ausgebildet, so daß eine zeitliche Höhenänderung des Oberflächenprofils der beiden Stirnflächen des Probenkörpers zu einer zeitlichen Längenänderung des Probenkörpers insgesamt führt. Die zeitliche Höhenänderung des Oberflächenprofils bzw. die sich hieraus ergebende Längenänderung des Probenkörpers wird verfahrensgemäß dazu verwandt, die Menge der an den Probenkörper abgegebenen Komponente zu bestimmen. Die gemessene Längenänderung kann dabei als direkt meßbare Größe dazu verwandt werden, die Komponentenmenge zu bestimmen, die vom Probenkörper aufgenommen wurde.

Gemäß einem weiteren bevorzugten Merkmal der Erfindung werden der Probenkörper und die Werkstücke der Wirkung der Gasatmosphäre bei gleicher Temperatur ausgesetzt. Erreicht wird hierdurch, daß sowohl für den Probenkörper als auch für die Werkstücke gleiche Anfangsbedingungen gelten, so daß ohne Korrektivrechnung die am Probenkörper gemessenen Verhältnisse auf die behandelten Werkstücke übertragen werden können. Dies gilt insbesondere, wenn für den Probenkörper eine mit den Werkstücken identische Legierung gewählt wird. Angeordnet werden kann der Probenkörper direkt im Ofenraum der thermochemischen Wärmebehandlungsanlage. Als in-situ-Sensor oder C-Strom-Sensor kann der Probenkörper mithin direkt in die thermochemische Behandlungsanlage eingebaut sein. Alternativ kann auch vorgesehen sein, den Probenkörper außerhalb der Behandlungsanlage anzuordnen und diesen mit einem aus der Behandlungsatmosphäre extrahierten Gasstrom zu beaufschlagen.

Gemäß einem weiteren Merkmal der Erfindung ist vorgesehen, daß die Verfahrensdurchführung isotherm erfolgt. Diese Verfahrensdurchführung hat den Vorteil, daß der Probenkörper einer von Temperaturen unbeeinflußten Längenänderung unterliegt. Die Bestimmung der Menge an aufgenommener Komponente kann dann auf einfache Weise ohne Korrekturrechnung durchgeführt werden. Alternativ hierzu kann auch vorgesehen sein, das Verfahren bei sich ändernden Temperaturen durchzuführen. Dies ist insbesondere dann erforderlich, wenn die Verfahrensdurchführung zur Behandlung der metallischen Werkstücke verfahrensbedingt nicht nur bei einer Temperatur erfolgen kann. Die Verfahrensdurchführung bei sich ändernden Temperaturen macht es allerdings erforderlich, die sich infolge der Temperaturänderungen zusätzlich ergebenden Längenänderungen des Probenkörpers rechnerisch zu kompensieren. Dies stellt zwar gegenüber einer isothermen Verfahrensdurchführung einen zusätzlichen Aufwand dar, eröffnet aber den Vorteil, das erfindungsgemäße Verfahren auch bei solchen Verfahrensdurchführungen anwenden zu können, bei denen sich während der Verfahrensdurchführung unterschiedliche Temperaturen einstellen.

Gemäß einem weiteren bevorzugten Merkmal der Erfindung ist vorgesehen, daß die Größe der bei einer definierten Abkühlung des Probenkörpers auftretenden Änderung des Oberflächenprofils, vorzugsweise der Längenänderung des Probenkörpers in Längsrichtung, und/oder der Temperaturbereich dieser Änderung zur Bestimmung der Menge und der Verteilung der übertragenen Komponente verwendet wird. Bekannterweise treten bei Phasenumwandlung von Eisenbasiswerkstoffen, zum Beispiel bei der Umwandlung von Austenit in Perlit, Volumensänderungen bzw. Längenänderungen auf, deren Größe und Temperaturbereich mit der Menge und Verteilung der aus der Gasphase vorgängig aufgenommenen Komponente zusammenhängen. Anders als die aus dem Stand der Technik bekannten Meß- und Auswertemethoden eröffnet das erfindungsgemäße Verfahren mithin die Möglichkeit, nicht nur die Menge, sondern auch die Verteilung der aus der Gasatmosphäre auf das Werkstück oder den Probenkörper abgegebenen Menge der Komponente zu bestimmen bzw. vorherzusagen. Insbesondere bei geometrisch aufwendigen Bauteilen ist dies von Vorteil, denn können auch exakte Vorhersagen bezüglich der Anreicherung der aus der Gasatmosphäre stammenden Komponenten am Bauteil auch für diejenigen Bereiche getroffen werden, die geometrisch schwer zugänglich sind. Auch läßt sich natürlich unter Verwendung des erfindungsgemäßen Verfahrens ein Konzentrationsprofil bestimmen bzw. vorhersagen.

Gemäß einem weiteren bevorzugten Merkmal der Erfindung ist vorgesehen, daß der Stoffübergang an dem Probenkörper analog zu dem an den Werkstücken stattfindet. Dies kann dadurch erreicht werden, daß der Probenkörper direkt im Ofenraum in der Nähe der Werkstücke angeordnet ist bzw. wenn er außerhalb des Ofenraums angeordnet ist, daß analoge Strömungs- und Temperaturverhältnisse am Probenkörper eingestellt werden.

Gemäß einem weiteren bevorzugten Merkmal der Erfindung strömt die die Komponente enthaltene Gasatmosphäre mit definierter Strömungsgeschwindigkeit am Probenkörper und/oder den Werkstücken vorbei. Diese Verfahrensvariation unterstützt in vorteilhafter Weise die Verfahrensdurchführung mit der Folge, daß eine vergleichsweise präzisere Messung der Längenänderung des Probenkörpers und damit auch eine genauere Angabe der Menge an übertragener Komponente vorgenommen werden kann.

Gemäß einem weiteren bevorzugten Merkmal der Erfindung erfolgt bei einer Verwendung eines rohrförmigen Probenkörpers die Aufnahme der Komponente nur über die äußere Mantelfläche. Erreicht werden kann dies beispielsweise dadurch, daß die innere Mantelfläche des rohrförmigen Probenkörpers abgedeckt oder mit einer für die Komponente undurchlässigen Deckschicht versehen ist.

Gemäß einem weiteren bevorzugten Merkmal der Erfindung ist vorgesehen, daß als Komponente Kohlenstoff verwendet wird. Der Probenkörper dient dann mithin als "C-Sensor" bzw. "C-Sonde" und ermöglicht die Bestimmung der Menge an Kohlenstoff, die bei einem Aufkohlungsvorgang aus einem der Gasatmosphäre zugegebenen Kohlenstoffträger an den Probenkörper und damit auch an die aufzukohlende Werkstücke übergeben wird.

Zur Durchführung des erfindungsgemäßen Verfahrens kann Verwendung finden, eine Vorrichtung zur kontinuierlichen Messung der bei einer thermischen Behandlung metallischer Werkstücke aus der die Werkstücke umgebenden Gasatmosphäre abgegebenen und von den Werkstücken aufgenommenen Menge einer Komponente, mit einer Aufnahme für einen Probenkörper, dessen Längserstreckung seine Quererstreckung wesentlich übersteigt, einem Längenmeßsystem zur Erfassung einer zeitlichen Längenänderung des Probenkörpers in Längsrichtung sowie einer Rechnereinheit.

Vorrichtungsseitig ist eine Aufnahme für den Probenkörper vorgesehen. Die Aufnahme dient der lagesicheren Fixierung des Probenkörpers innerhalb der Gasatmosphäre. Angeschlossen an diese Aufnahme ist ein Längenmeßsystem, das der Erfassung einer zeitlichen Längenänderung des Probenkörpers innerhalb der Aufnahme dient. Gemessen wird dabei die Längenänderung des Probenkörpers in Längsrichtung. Die vom Längenmeßsystem erfaßte Längenänderung wird einer Rechnereinheit zugeführt, die unter Verwendung vorgegebener Rechenschritte aus der gemessenen Längenänderung die Menge an aus der Gasatmosphäre in den Probenkörper übertragener Komponente bestimmt. Die Verwendung der erfindungsgemäßen Vorrichtung erlaubt es in vorteilhafter Weise, anhand einer direkt meßbaren geometrischen Größe des Probenkörpers die Menge an übertragener Komponente zu bestimmen, so daß unter Verwendung der erfindungsgemäßen Vorrichtung Störgrößen, die bei den aus dem Stand der Technik bekannten indirekten Meß- und Auswertemethoden einer Verfälschung des Meßergebnisses bewirken, weitestgehend eliminiert werden können. Unter Verwendung der erfindungsgemäßen Vorrichtung lassen sich somit exakte Meßgrößen und somit auch exakte Vorhersagen in Bezug auf die vom Probenkörper bzw. den Werkstücken aus der Gasatmosphäre aufgenommene Menge einer Komponente machen.

Es kann vorrichtungsseitig fernervorgesehen sein, daß die Aufnahme für den Probenkörper zwei Widerlager aufweist, wobei das eine Widerlager in seiner Position zum Längenmeßsystem fixiert ist. Vorgesehen ist mithin ein erstes zum Längenmeßsystem nicht verschieblich angeordnetes Widerlager sowie ein zweites Widerlager, das sowohl zum ersten Widerlager als auch zum Längenmeßsystem relativ verschieblich angeordnet ist. Ein von der Aufnahme aufgenommener Probenkörper wird stirnseitig von den beiden Widerlagern gehalten. Zu Beginn der thermochemischen Verfahrensdurchführung sind die Stirnseiten des Probenkörpers eben ausgeführt und liegen flächig an ihren jeweiligen Widerlagern an. Im Zuge der Verfahrensdurchführung kommt es durch Aufnahme der aus der Gasatmosphäre stammenden Komponente zu einer Volumensänderung des Probenkörpers, was auch eine Änderung des Oberflächenprofils an den beiden an den Widerlagern anliegenden Stirnflächen des Probenkörpers führt. Die Änderung des Oberflächenprofils drückt sich durch eine zeitliche Höhenänderung der anfänglich eben ausgebildeten Stirnflächen aus, so daß es infolge der Anreicherung der aus der Gasatmosphäre stammende Komponente im Probenkörper zu einer Ausdehnung des Probenkörpers auch in Längsrichtung kommt. Bedingt durch diese Längenänderung in Längsrichtung wird das relativ verschieblich angeordnete Widerlager verschoben. Dieser Widerlagerverschub wird vom Längenmeßsystem erfaßt und an die Rechnereinheit übertragen, die dann auf Basis einer vorgebbaren Rechenregel die erfaßte Längenänderung des Probenkörpers zur Ermittlung der Menge an aufgenommener Komponente umsetzt.

Es ist vorzugsweise wenigstens ein Teil der Aufnahme für den Probenkörper innerhalb der Gasatmosphäre angeordnet. Sichergestellt wird durch diese Maßnahme, daß der Probenkörper weitestgehend der gleichen Gasatmosphäre ausgesetzt ist wie auch die zu behandelnden Werkstücke. Vorzugsweise ist deshalb vorzusehen, daß die gesamte Aufnahme samt Probenkörper innerhalb der Gasatmosphäre angeordnet ist.

Das Längenmeßsystem ist vorzugsweise außerhalb der Gasatmosphäre bzw. zumindestens außerhalb des erhitzten Bereichs der Gasatmosphäre angeordnet. Eine solche Anordnung hat den Vorteil, daß dieses nicht der innerhalb der Gasatmosphäre herrschenden Temperatur bzw. dem dort herrschenden Druck ausgesetzt ist. Durch äußere Einflüsse bedingte Verfälschungen des Meßergebnisses können so in vorteilhafter Weise ausgeschlossen werden.

Das Längenmeßsystem ist vorzugsweise ein optisch arbeitendes Längenmeßsystem. Vorzugsweise wird ein mittels Laser arbeitendes Längenmeßsystem verwendet, welches insbesondere präzise Meßergebnisse liefert. Alternativ kann auch vorgesehen sein, ein mechanisch arbeitendes Längenmeßsystem zu verwenden, was im Unterschied zu einem optisch arbeitenden Längenmeßsystem den Vorteil hat, weniger störanfällig gegenüber äußeren Einflüssen zu sein.

Es ist vorzugsweise ein Strömungskanal für den Probenkörper vorgesehen. Verwendet werden kann als Strömungskanal beispielsweise ein Rohr, innerhalb dem ein Probenkörper angeordnet ist. Die Verwendung eines Strömungskanals hat dabei den Vorteil, daß eine für den Probekörper definierte Strömungsumgebung geschaffen werden kann, was die Bestimmung eines genaueren Meßergebnisses ermöglicht.

Es ist vorzugsweise ein Temperaturfühler vorgesehen. Dieser dient der Bestimmung der sich im Laufe der Verfahrensdurchführung unter Umständen ändernden Temperatur innerhalb der die Werkstücke und den Probenkörper umgebenden Atmosphäre. Die sich infolge einer Temperaturänderung zusätzlich ergebenden Längenänderungen des Probenkörpers können so anhand der ermittelten Temperaturwerte erfaßt und zur Durchführung einer Korrekturrechnung an die angeschlossene Recheneinheit übertragen werden. Die Bestimmung der Menge an übergegangener Komponente ergibt sich dann aus der gemessenen Längenänderung des Probenkörpers einerseits sowie der durch die Temperaturänderung bestimmten zusätzlichen Längenänderung des Probenkörpers andererseits.

Als Probenkörper wird vorzugsweise verwendet ein Probenkörper zur kontinuierlichen Messung der bei einer thermochemischen Behandlung metallischer Werkstücke aus der die Werkstücke umgebenden Gasatmosphäre abgegebenen und von den Werkstücken aufgenommenen Menge einer Komponente mit einer die Quererstreckung im wesentlichen übersteigenden Längserstreckung.

Eine solche geometrische Ausgestaltung des Probenkörpers hat den Vorteil, daß die infolge der aus der Gasatmosphäre aufgenommenen Komponente bewirkte Volumensänderung sich insbesondere im Hinblick auf die Längsausdehnung bemerkbar macht, was eine vereinfachte Verfahrensdurchführung in vorteilhafter Weise zur Folge hat. Verwendet werden können als Probenkörper vorzugsweise ein Rundstab oder ein als Rohr ausgebildeter Probenkörper. Um zu verhindern, daß bei einem rohrförmig ausgestalteten Probenkörper die innere Mantelfläche die aus der Gasatmosphäre stammende Komponente aufnimmt, kann diese bedarfsweise mit einer für die aus der Atmosphäre stammende Komponente undurchlässigen Deckschicht versehen sein.

Weitere Vorteile und Merkmale der Erfindung ergeben sich anhand der Beschreibung der nachfolgenden Figuren. Dabei zeigen:
- Fig. 1:: ein Diagramm des Verlaufes des Konzentrationsprofils;
- Fig. 2:: ein Diagramm des Verlaufes des Längenprofils;
- Fig. 3:: unter der Annahme eines plastischen Verhaltens ein Diagramm des Verlaufes des Konzentrationsprofils zu einem Zeitpunkt t1;
- Fig. 4:: unter der Annahme eines plastischen Verhaltens ein Diagramm des Verlaufes des Konzentrationsprofils zu einem Zeitpunkt t2;
- Fig. 5:: ein Diagramm des Verlaufes des Konzentrationsprofils gemäß einem ersten Verfahrensschritt;
- Fig. 6:: ein Diagramm des Verlaufes des Konzentrationsprofils gemäß einem zweiten Verfahrensschritt;
- Fig. 7:: in einer schematischen Darstellung die erfindungsgemäße Vorrichtung gemäß einem ersten Ausführungsbeispiel;
- Fig. 8:: in einer schematischen Darstellung die erfindungsgemäße Vorrichtung gemäß einem zweiten Ausführungsbeispiel;
- Fig. 9:: ein Diagramm der zeitlichen Längenänderung eines Probenkörpers;
- Fig. 10:: ein Diagramm des Verlaufes der Längenänderung über der Temperatur und
- Fig. 11:: in einer schematischen Darstellung die erfindungsgemäße Vorrichtung gemäße einer dritten Ausführungsform.

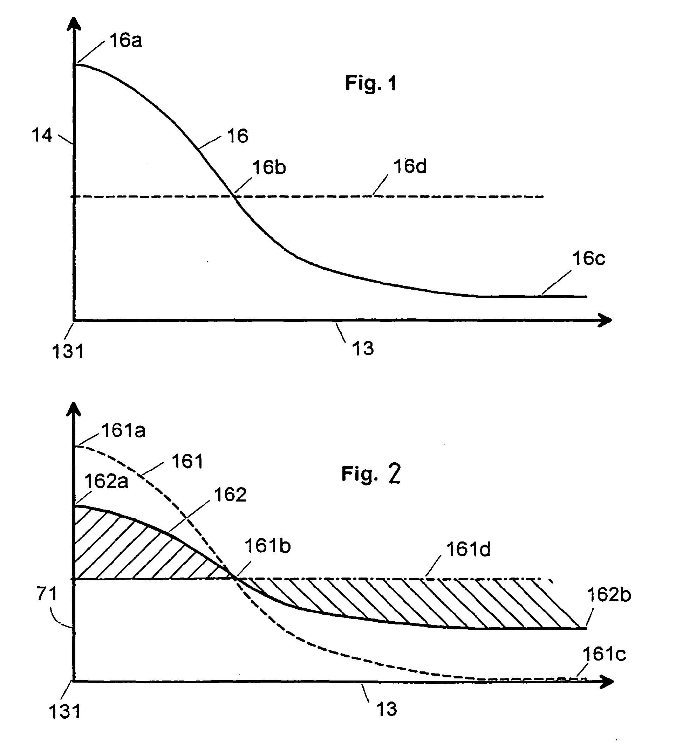

Fig. 1 zeigt in Form eines Graphens den Verlauf des Konzentrationsprofils 16 eines Probenkörpers in einem Schnitt senkrecht zur Längsrichtung des Probenkörpers. Dabei bezeichnet 13 die Ortskoordinate und 14 die Konzentrationskoordinate. Der Schnittpunkt von Ortskoordinate 13 und Konzentrationskoordinate 14 ist mit 131 bezeichnet und entspricht in Bezug auf die Ortskoordinate 13 dem Rand, d. h. der Mantelfläche des Probenkörpers.

Wie dem Verlauf des Konzentrationsprofils 16 deutlich zu entnehmen ist, geht das Profil von der maximalen Randkonzentration 16a aus und fällt mit fortschreitender Ortskoordinate 13 bis auf den Wert 16c ab, der im wesentlichen der Anfangskonzentration des Probenkörpers vor der Durchführung des thermochemischen Prozesses entspricht. Der Punkt 16b bezeichnet den Schnittpunkt zwischen der Verlaufskurve des Konzentrationsprofils 16 und den Mittelwert 16d des Konzentrationsprofils 16 bezogen auf die Randkonzentration 16a und die Anfangskonzentration 16c.

Im Rahmen der Durchführung eines thermochemischen Prozesses zur Behandlung metallischer Werkstücke geht eine Komponente, beispielsweise Kohlenstoff aus der Gasatmosphäre auf das metallische Werkstück über. Die Einbringung von Atomen auf Zwischengitterplätze in dem Metallgitter des Werkstückes führt dabei zu einer Gitteraufweitung, welche auf makroskopischer Ebene zu einer Volumenszunahme führt. Diese Volumenszunahme erfolgt näherungsweise proportional zur aufgenommenen Konzentration. An einem Probenkörper, dessen Längserstreckung im wesentlichen die Quererstreckung übersteigt, kann diese Volumenszunahme als lineare Verlängerung in Längsrichtung wahrgenommen werden. Wird dieser Längenveränderung mit der Ausgangslänge verglichen, so ergibt sich hieraus eine relative Verlängerung des Probenkörpers in Funktion der zugeführten Konzentrationsveränderung.

Fig. 2 zeigt unter der Annahme eines linear-elastischen Verhaltens des Probenkörpers gemäß Kurve 161 das Längenprofil des Probenkörpers entlang der Ortskoordinate 13, wie es sich bei einem Probenkörper unter der Annahme einstellen würde, daß seine einzelnen Schichten mechanisch frei gegeneinander beweglich wären. Aufgrund der Proportionalität zwischen relativer Verlängerung einerseits und Komponentenaufnahme andererseits ergibt sich ein Längenprofil 161, das proportional zum Konzentrationsprofil 162 verläuft. Am Rand, d.h. im äußeren Bereich der Mantelfläche des Probenkörpers stellt sich dabei eine maximale relative Länge 161a ein, wohingegen im Inneren des Probenkörpers die Ausgangslänge 161c unverändert beibehalten bleibt. Die strichpunktierte Gerade 161d entspricht dem Mittelwert der relativen Längenänderung.

Der in Fig. 2 gezeigte Verlauf geht von der hypothetischen Annahme aus, die einzelnen Schichten des Probenkörpers seien mechanisch frei gegeneinander beweglich. In der Realität sind die einzelnen Schichten jedoch miteinander verbunden, d. h. sie können sich nicht frei entsprechend der lokalen Konzentration einer aufgenommenen Komponente in der Länge einstellen, so daß Schubspannungen entstehen. Der in Fig. 2 mit 161b bezeichnete Ort identifiziert die neutrale Faser, d. h. diejenige Linie des Probenkörpers, bei der die Druckspannungen des höher angereicherten Randbereichs in die Zugspannungen des Probenkörperinneren übergehen. Durch die Wirkung dieser Schubspannungen wird das hypothetisch in Fig. 2 dargestellte Profil der relativen Längenänderung 161 zum effektiven Profil der Längenänderung 162 verformt, d. h. die maximale Verlängerung 161a wird unter dem Einfluß der Druckspannungen zum Verlängerungswert 162a reduziert, während die minimale relative Verlängerung 161c durch die Zugspannung bis zum Wert 162b angehoben wird.

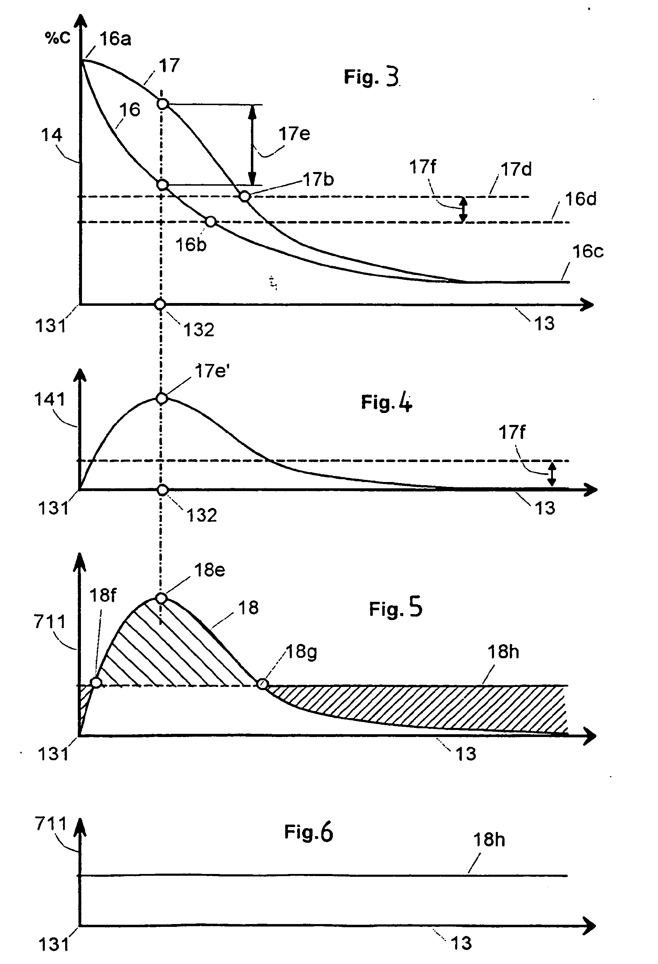

Mit Bezug auf die Figuren 3 bis 4 wird ein vollplastisches Verhalten angenommen. Dabei zeigt Bild 3 das Konzentrationsprofil 16 zu einem Zeitpunkt t1 und das Konzentrationsprofil 17 zu einem späteren Zeitpunkt t2. 16a bezeichnet die konstante Randkonzentration des Profils und 16c entspricht dem Anfangswert vor Beginn der Eindiffusion, welcher im Inneren des Probenkörpers zu diesen beiden Zeitpunkten gleichsam vorhanden ist. 16b und 17b bezeichnet jeweils den Ort, an welchem sich die mittlere Konzentration, entsprechend den gestrichelten Graphen 16d und 17d zu den beiden Zeitpunkten eingestellt hat. Die Koordinate 132 entspricht dem Ort der maximalen Konzentrationsänderung 17e zwischen den beiden Zeitpunkten t1 und t2.

Das Diagramm gemäß Fig. 4 zeigt die lokale Konzentrationsänderung entsprechend der Achse 141 zwischen den beiden Zeitpunkten t1 und t2 entlang der Ortskoordinatenachse 13, d. h. in Diffusionsrichtung quer zur Längsrichtung des Probenkörpers. Dabei entspricht die gestrichelte Linie 17f der Zunahme der mittleren Konzentration zwischen diesen Zeitpunkten.

Für das Verständnis des Diagramms gemäß Fig. 5, in dem die Achse 711 die relative Längenänderung bezeichnet, wird angenommen, daß das Ausgangsprofil der Probenlänge zum Zeitpunkt t1 eben ist, d. h. ungleich dem Beispiel, welches im vollelastischen Fall der Figuren 1 und 2 gezeigt wurde, wird das Profil unter dem Einfluß der Schubspannungen laufend während der Eindiffusion eingeebnet. Würde dieser Relaxationsprozeß zwischen den Zeitpunkten t1 und t2 momentan "eingefroren", so ergäbe sich ein Profil der Längenveränderung entsprechend der Kurve 18 gemäß Fig. 5, wobei beim Koordinatenwert 132 die maximale relative Längenänderung 18e eintritt, welche zur Ausbildung einer Druckspannungszone führt. An den Punkten 18g und 18h geht die negative Schubspannung beidseitig in die Zonen der positiven Schubspannung über. Die Zone der Druckspannung sowie die beiden Zugspannungszonen sind in verschiedener Schraffur dargestellt.

Das Diagramm gemäß Fig. 6 zeigt den zweiten Gedankenschritt der Relaxation des relativen Längenprofils 18 gemäß Fig. 5. Dabei werden die unter Druckspannung stehenden Bereiche des Profils abgesenkt, während die anderen Bereiche angehoben werden, wobei ein gemeinsames Endniveau entsprechend dem Mittelwert 18h des Längenprofils über die gemeinsame Fläche eingestellt wird.

In der Realität erfolgen die in den Figuren 5 und 6 gedanklich voneinander getrennten Verfahrensschritte gleichzeitig, d. h. die Relaxation überlagert die differenzielle örtliche Längenänderung des Profils. Als Resultat dieses Prozesses ergibt sich dann ein gleichmäßiges Längenwachstum der Probe, welches proportional zur Gesamthaft aufgenommenen Komponentenmenge bzw. zum Mittelwert des Konzentrationsprofils zu jenem Zeitpunkt ist. In der Praxis liegt die Änderung des stirnseitigen Profils des Probenkörpers bzw. seine Längenänderung bei thermochemischen Prozessen, welche wie die Aufkohlung bei hohen Temperaturen ablaufen, nahe beim vollplastischen Verhalten. Bei thermochemischen Prozessen, die bei tieferen Temperaturen ablaufen, wie zum Beispiel das Nitrieren, steigt der Anteil des elastischen Verhaltens.

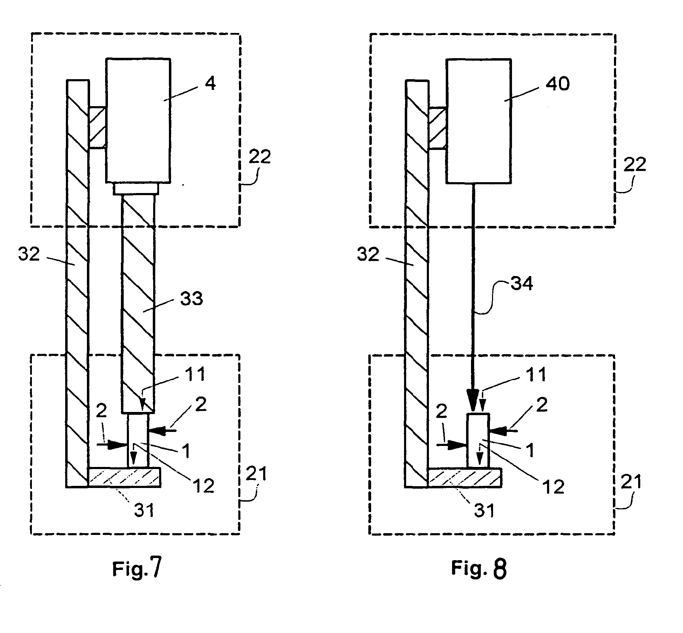

Fig. 7 zeigt beispielhaft eine Vorrichtung zur Durchführung des erfindungsgemäßen Verfahrens unter Verwendung einer mechanischen Längenmeßvorrichtung 4. Dabei befindet sich der Probenkörper 1 unter der Atmosphäre und bei der Temperatur des thermochemischen Prozesses in einer Zone 21. Die Gasatmosphäre, welche die Komponente abgibt, wirkt aus allen Richtungen entsprechend der Pfeile 2 auf die Mantelfläche der Probe 1 ein. Mit ihrer unteren Stirnfläche 12 liegt die Probe auf einer Widerlagerplatte 31 auf, die über eine Halterung 32 mit der Längenmeßvorrichtung 4 derart verbunden ist, daß eine relative Verschieblichkeit von Widerlagerplatte 31 und Längenmeßvorrichtung 4 unterbunden ist. Im Zuge der Komponenteneindifusion erfolgt eine Längenänderung des Probenkörpers 1 in Längsrichtung. Die Längenänderung wird über die höchsten Punkte des Oberflächenprofils der Stirnseiten 11 und 12 an die Stange 33 weitergegeben, welche diese an die Längenmeßvorrichtung 4 weitergibt.

Bei der Längenmeßvorrichtung 4 kann es sich beispielsweise um ein kapazitives oder ein induktives Meßsystem handeln, welches die Meßwerte an ein elektronisches und in den Figuren nicht dargestelltes Datenverarbeitungssystem übermittelt. Die Längenmaßvorrichtung 4 ist innerhalb einer Zone 22 angeordnet, die außerhalb der Zone 21 liegt.

Fig. 8 zeigt eine entsprechende Anordnung der erfindungsgemäßen Vorrichtung unter Verwendung einer optisch arbeitenden Längenmeßvorrichtung 40. Dabei sind im Vergleich zu Fig. 7 baugleiche Teile mit gleichen Nummern versehen. Der Pfeil 34 entspricht dabei der Meßstange 33 gemäß Fig. 7 und stellt den Meßstrahl des optischen Systems dar, der beispielsweise als Laserstrahl ausgebildet sein kann.

Eine weitere Meßmethode zur Erfassung der Längenänderung des Probenkörpers 1 ergibt sich durch die Verwendung von Dehnungsmeßstreifen. Diese werden in Längsrichtung des Probenkörpers auf diesen aufgebracht, die dann die Längenveränderung der Mantelfläche des Probenkörpers gleichfalls mitvollziehen. Der temperaturkompensierte Dehnungswert kann dann ebenfalls an einer Datenverarbeitung zur Auswertung der gemessenen Längenänderung weitergegeben werden.

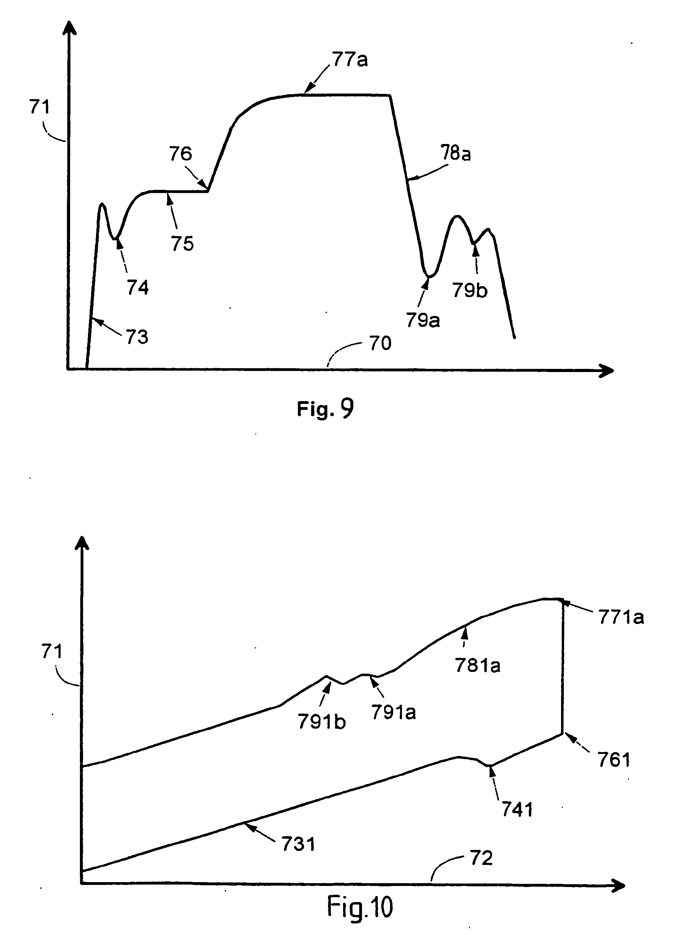

Fig. 9 zeigt ein typisches Diagramm der zeitlichen Längenänderung eines Probenkörpers, wie sie z. B. mit einer Verfahrensanordnung entsprechend Fig. 7 bei der Aufkohlung einer Probe des Stahls Ck15 gemessen wird. Dabei repräsentiert der Pfeil 70 die Zeitachse und der Pfeil 71 die Achse der Längenänderung. Das Kurvenstück 73 bezeichnet die Längenänderung, welche beim Aufheizvorgang unter einer inerten Gasatmosphäre auftritt und an die sich die Umwandlung des Ausgangsgefüges anschließt, wobei gemäß Punkt 74 das Gefüge in Austenit umwandelt. Das Kurvenstück 75 entspricht der Ausdehnung bis zur Temperaturkonstanz. Bis zu diesem Moment entspricht die Ausdehnungskurve einer üblichen dilatometrischen Meßkurve.

Beim Punkt 76 wird die inerte Gasatmosphäre durch eine aufkohlende Atmosphäre, z. B. ein Gemisch aus Kohlenmonoxid, Wasserstoff und Propan ersetzt. Augenblicklich zeigt sich eine Verlängerung der Probe, welche bei ausreichend langer Behandlungszeit in die Sättigung des Kurvenstücks 77a übergeht. In diesem Zusammenstand befindet sich die Konzentration im gesamten Probenkörper im Gleichgewicht mit der Kohlenstoffaktivität der Gasatmosphäre. Im dargestellten Beispiel wurde angenommen, daß diese Sättigung nahe der Löslichkeitsgrenze für Kohlenstoff im Austenit, d.h. bei der Linie Azm des metastabilen Eisen-Kohlenstoff-Diagramms liegt.

Das Kurvenstück 78a entspricht der Abkühlung der Probe, wobei am Punkt 79a die Ausscheidung von Sekundärzementit und beim Punkt 79b die Ausscheidung von Perlit einsetzt. Im vorliegenden Beispiel wurde angenommen, daß der Sättigungswert im übereutektoiden Bereich liegt. Liegt die Sättigung des Probenkörpers hingegen im untereutektoiden Bereich, dann würde Punkt 79a der Ausscheidung von Ferrit entsprechen.

Bei Aufkohlungsversuchen, welche nicht bis um Sättigungsgleichgewicht mit der Gasatmosphäre geführt werden, indem die aufkohlende Gasatmosphäre z. B. durch Inertgase ersetzt wird, bricht das Kurvenstück nach Punkt 76 zur Erreichung des Sättigungsplateaus 77a plötzlich ab und geht fast unmittelbar in eine waagerechte Linie über. Da zu diesem Zeitpunkt in der Probe ein von außen nach innen abfallendes Profil vorliegt, entspricht die Längenänderung der Kurve, wie sie bei der Abkühlung analog zu den Punkten 79a und 79b der Überlagerung der Umwandlungen verschiedener kohlenstoffhaltiger Legierungen entsteht.

Fig. 10 zeigt die oben beschriebene Versuche in einem Diagramm der Längenänderung gegen die Temperatur. Dabei repräsentieren der Pfeil 72 die Temperaturachse und der Pfeil 71 die relative Längenänderung.

Der Kurvenbereich 731 bis 761 repräsentiert die Aufheizung der Probe in inerter Atmosphäre, wobei die Umwandlung zum Austenit beim Punkt 741 abgeschlossen ist. Zwischen den Punkten 761 und 771a erfolgt anschließend die isotherme Längenänderung, welche auf den Aufkohlungsprozeß zurückgeht. Das Kurvenstück 771a - 781a - 791a - 791b entspricht der Abkühlung des Probenkörpers nach der Aufkohlung mit den Umwandlungen bei den Punkten 791a und 791b.

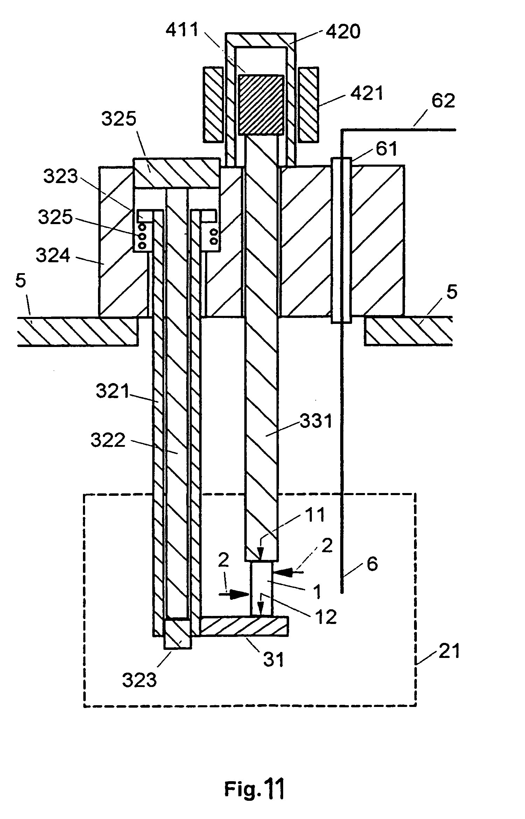

Fig. 11 zeigt eine beispielhafte Vorrichtung zur Messung der Längenänderung eines Probenkörpers entsprechend dem erfindungsgemäßen Verfahren. Dabei steht der Probenkörper 1 mit einer Stirnfläche 12 auf einer Widerlagerplatte 31 und stößt mit der anderen Stirnfläche 11 gegen eine Stange 331, die in Bezug auf den Probenkörper 1 ein zweites Widerlager darstellt. Der Probenkörper 1 ist während des thermochemischen Prozesses hohen Temperaturen ausgesetzt, wohingegen die eigentliche Längenmeßvorrichtung, welche wahlweise auf einem gekühlten Flansch 324 montiert sein kann, der seinerseits mit der Wand 5 gasdicht zum thermochemischen Reaktor abschließt, im wesentlichen einem Temperaturniveau nahe der Raumtemperatur ausgesetzt ist, entsteht ein steiler Temperaturgradient. Die Längenänderungen des Probenkörpers werden deshalb durch die Längenänderungen der Stange 331 sowie der Halterung der Widerlagerplatte 31 überlagert. Um diese Störwirkungen gering zu halten, wird die Stange 331 aus Keramik gefertigt, z. B. aus Quarz. Dabei ist es von großer Bedeutung, daß die Stirnfläche des Quarzstabes, welche mit der Stirnfläche 11 der Probe in Kontakt steht, völlig plangeschliffen ist und daß der Durchmesser denjenigen der Probe übersteigt, um sicherzustellen, daß alle Punkte der Stirnfläche 11 mit der Stirnfläche des Quarzstabes in Kontakt stehen.

Um die zwar geringen, jedoch infolge ihrer Längen immer noch vorhandenen Längenänderungen der Stange 331 im Temperaturgradient zu kompensieren, wird der Abstand der Widerlagerplatte 31 gegenüber dem Flansch 324 ebenfalls durch eine Stange 322, welche bevorzugt aus demselben keramischen Material besteht wie die Stange 331, eingestellt. Da keramische Materialien sich bei mechanischer Belastung spröde verhalten, wird die Widerlagerplatte 31 an einem hochtemperaturbeständigen Metallrohr 321 angeordnet, welches mit dem Metallring 323 gegen eine in einer Bohrung 325 des Blockes 324 befindliche Druckfeder 325 drückt. Die keramische Stange 322, welche unten über das Metallrohr 321 fest mit der Widerlagerplatte 31 verbunden ist, wird dadurch gegen den stirnseitigen mit dem Block 324 verbundenen Metallbolzen angepaßt. Die bei Temperaturänderung große Ausdehnung des Metallrohres 321 wirkt sich daher nur auf die veränderte Pressung der Feder 325 aus, während der Abstand der Widerlagerplattform 31 zum Metallblock 324 durch die weniger veränderter Länge der keramischen Stange 322 bestimmt wird.

Die eigentliche Längenmessung erfolgt im Beispiel gemäß Fig. 11 induktiv, indem die Keramikstange 331 oben einen zylindrischen Metallkörper 411 besitzt, dessen Verschiebung von der induktiven Meßspule 421 durch die Wand 420 wahrgenommen wird.

Vorgesehen ist des weiteren ein Temperaturfühler 6, wie z. B. ein Mantelthermoelement, das über eine Durchführung 61 durch den Flansch 324 geleitet und über die Verbindung 62 an die Datenerfassung angeschlossen ist. Die Meßwerte sowohl der Längenmeßvorrichtung als auch des Temperaturfühlers werden einer in der Figur nicht dargestellten Datenerfassung zugeleitet und anschließend mit einem entsprechenden elektronischen Rechenprogramm in ein Konzentrationsprofil der Komponente im Probenkörper umgerechnet.

Ein wesentlicher Vorteil des Verfahrens ist der Umstand, daß die Diffusionsgeometrie in weiten Grenzen verändert und damit die Aktivität und Zeitdauer des zu kontrollierenden thermochemischen Prozesses angepaßt werden kann, z. B. von einem Rohr mit einer Wandstärke von 0,1 mm bis hin zu einem zylindrischen Vollkörper mit einem Durchmesser von 6 mm und mehr. Zusätzlich kann auch die Legierung des Probenkörpers in Übereinstimmung mit dem Material der zu kontrollierenden Chargen gewählt werden, so daß die Resultate der Messung direkt auf das Verhalten der Charge übertragen werden können.

Am Beispiel des Einsatzhärtungsprozesses soll nachfolgend die Durchführung des erfindungsgemäßen Kontrollverfahrens aufgezeigt werden:

Bei kürzeren Aufkohlungszeiten kann weitgehend unabhängig von der Form des Werkstücks ein Prozeß der eindimensionalen nichtstationären Diffusion angenommen werden. Bei ausreichend großer Wandstärke des Probenkörpers, z. B. einem Vollzylinder mit ausreichend großem Durchmesser, gilt dies auch für den Probenkörper. Der Diffusionskoeffizient für ein bestimmtes Material ist bei bekannter Temperatur eine Materialkonstante, mit der sich bei bekannter Kohlenstoff-Randkonzentration das Diffusionsprofil einfach errechnen läßt. Die Randkonzentration ist im Gleichgewicht eine Funktion der Kohlenstoffaktivität der Gasatmosphäre, welche aus der Gaszusammensetzung errechnet und über die Löslichkeitscharakteristik des Stahls unter Benutzung der Aktivitätskoeffizienten der Legierung in eine Kohlenstoffkonzentration umgerechnet werden kann. Jedoch ist die Größe des Stoffübergangskoeffizienten des Kohlenstoffes von der Gasatmosphäre in die Oberfläche des Metallgitters nicht bekannt. Das Profil kann nun wie folgt berechnet werden: Aufgrund des erfindungsgemäßen Verfahrens ist zu jedem Zeitpunkt die Menge an Kohlenstoff bekannt, welche gesamthaft in den Versuchskörper eingedrungen ist. Daraus ergibt sich direkt der auf die Zeit- und Flächeneinheit bezogene Kohlenstoff-Massenstrom. Die erfindungsgemäße Vorrichtung ist somit ein direkt messender C-Strom-Sensor. Aufgrund bekannter Diffusionsdaten wird über die Arrhenius-Gleichung der Diffusionskoeffizient des Kohlenstoffs in der Legierung für die verwendete Temperatur errechnet. Mit Hilfe einer Lösung der nichtstationären Diffusionsgleichung für Zylinderkoordinaten wird die Randkonzentration bestimmt, welche mit dem bekannten Diffusionskoeffizienten in der abgelaufen Zeit zum gemessenen mittleren Kohlenstoffgehalt führt. Diese Randkonzentration wird über die nicht stationäre Gleichung in ein Konzentrationsprofil im Rahmen der Geometrie des Werkstückes umgesetzt.

Die oben für den Fall der Einsatzhärtung beschriebenen Vorgänge können sinngemäß auch für andere thermochemische Prozesse z. B. den Aufstickungsprozeß angewendet werden.

Wird im Rahmen eines Aufkohlungsprozesses die Temperatur verändert, so kann die Änderung des Kohlenstoffgehalts trotzdem exakt bestimmt werden. Zu diesem Zweck wird die Längenänderung, welche sich im betrachteten Temperaturbereich aufgrund des linearen Ausdehnungskoeffizienten des Austenits ergibt, von der gesamten Längenänderung abgezogen. Der Restbetrag der Längenänderung entspricht der relativen Menge des vom Probenkörper aufgenommen Kohlenstoffs.

## Patentansprüche

1. Verfahren zur kontinuierlichen Messung der bei einer thermochemischen Behandlung metallischer Werkstücke aus der die Werkstücke umgebenden Gasatmosphäre abgegebenen und von den Werkstücken aufgenommenen Menge einer Komponente, bei dem ein Probenkörper, dessen Längserstreckung seine Quererstreckung wesentlich übersteigt, der Wirkung der Gasatmosphäre ausgesetzt, die infolge der Aufnahme der aus der Gasatmosphäre abgegebenen Komponente verursachte zeitliche Längenänderung des Probenkörpers in Längsrichtung gemessen und die gemessene Längenänderung zur Bestimmung der Menge der aus der Gasatmosphäre in den Probenkörper übertragenen Komponente verwendet wird.

2. Verfahren nach Anspruch 1, **dadurch gekennzeichnet, daß** der Probenkörper und die Werkstücke der Wirkung der Gasatmosphäre bei gleicher Temperatur ausgesetzt werden.

3. Verfahren nach Anspruch 1 oder 2, **dadurch gekennzeichnet, daß** dieses isotherm durchgeführt wird.

4. Verfahren nach Anspruch 1 oder 2, **dadurch gekennzeichnet, daß** dieses bei sich ändernden Temperaturen durchgeführt wird, wobei sich infolge der Temperaturänderungen zusätzlich ergebenden Längenänderungen des Probenkörpers rechnerisch kompensiert werden.

5. Verfahren nach einem der vorhergehenden Ansprüche, **dadurch gekennzeichnet, daß** die Größe der bei einer definierten Abkühlung des Probenkörpers auftretenden Änderung des Oberflächenprofils, vorzugsweise der Längenänderung des Probenkörpers in Längsrichtung, und/oder der Temperaturbereich dieser Änderung zur Bestimmung der Menge und der Verteilung der übertragenen Komponente verwendet wird.

6. Verfahren nach einem der vorhergehenden Ansprüche, **dadurch gekennzeichnet, daß** bei der Verwendung eines rohrförmigen Probenkörpers die Aufnahme der Komponente nur über die äußere Mantelfläche erfolgt.

7. Verfahren nach einem der Ansprüche 1 bis 5, **dadurch gekennzeichnet, daß** bei der Verwendung eines rohrförmigen Probenkörpers die Aufnahme der Komponente über die äußere und die innere Mantelfläche erfolgt.

8. Verfahren nach einem der vorhergehenden Ansprüche, **dadurch gekennzeichnet, daß** als Komponente Kohlenstoff verwendet wird.

## Claims

1. A method for continuously measuring the quantity of a component emitted from the gaseous atmosphere surrounding metal work pieces and absorbed by the metal work pieces during the thermochemical treatment of the metal work pieces, wherein, given a specimen with a longitudinal extension significantly exceeding its transverse extension that is exposed to the gaseous atmosphere, the change in length of the specimen in the longitudinal direction caused by the absorption of the component emitted by the gaseous atmosphere is measured, and the measured change in length is used to determine the quantity of component transferred from the gaseous atmosphere into the specimen.

2. The method according to claim 1, **characterized in that** the specimen and the work pieces are exposed to the gas atmosphere at the same temperature.

3. The method according to claim 1 or 2, **characterized in that** it is performed isothermally.

4. The method according to claim 1 or 2, **characterized in that** it is performed at changing temperatures, wherein additionally arising changes in lengths of the specimen caused by the temperature changes are offset by calculation.

5. The method according to one of the preceding claims, **characterized in that** the size of the change in surface profile arising during a defined cooling of the specimen, preferably the change in length of the specimen in the longitudinal direction, and/or the temperature range of this change is used to determine the quantity and distribution of the transferred component.

6. The method according to one of the preceding claims, **characterized in that** the component is only absorbed via the outer jacket surface when using a tubular specimen.

7. The method according to one of claims 1 to 5,
**characterized in that** the component is absorbed via the outer and inner jacket surface when using a tubular specimen.

8. The method according to one of the preceding claims, **characterized in that** carbon is used as the component.

## Revendications

1. Procédé pour la mesure continue de la quantité d'un composant dégagé par l'atmosphère gazeuse entourant des pièces usinées lors d'un traitement thermochimique de pièces usinées métalliques et admis par les pièces usinées, dans lequel un corps d'échantillon, dont l'étendue longitudinale dépasse considérablement son étendue transversale, est exposé à l'effet de l'atmosphère gazeuse, la modification dans le temps de la longueur du corps d'échantillon, résultant de l'admission des composants dégagés par l'atmosphère gazeuse, est mesurée dans le sens longitudinal, et la modification de longueur mesurée est utilisée pour la détermination de la quantité des composants transmis par l'atmosphère gazeuse dans le corps d'échantillon.

2. Procédé selon la revendication 1, **caractérisé en ce que** le corps d'échantillon et les pièces usinées sont exposés à l'effet de l'atmosphère gazeuse, à une température identique.

3. Procédé selon l'une des revendications 1 ou 2,
**caractérisé en ce que** cela est réalisé de façon isotherme.

4. Procédé selon l'une des revendications 1 ou 2,
**caractérisé en ce que** cela est réalisé à des températures variables, les modifications de longueur supplémentaires du corps d'échantillon, résultant des variations de température, sont compensées par voie de calcul.

5. Procédé selon l'une des revendications précédentes, **caractérisé en ce que** la grandeur de la modification du profil de surface, de préférence la grandeur de la modification de la longueur du corps d'échantillon dans le sens longitudinal, apparaissant lors d'un refroidissement défini du corps d'échantillon, et/ou la plage de température de cette modification sont utilisées pour la détermination de la quantité et de la répartition des composants transmis.

6. Procédé selon l'une des revendications précédentes, **caractérisé en ce que** lors de l'utilisation d'un corps d'échantillon en forme de tuyau, l'admission des composants ne se fait que par la surface d'enveloppe extérieure.

7. Procédé selon l'une des revendications 1 à 5,
**caractérisé en ce que** lors de l'utilisation d'un corps d'échantillon en forme de tuyau, l'admission des composants se fait par la surface d'enveloppe extérieure et intérieure.

8. Procédé selon l'une des revendications précédentes, **caractérisé en ce que** les composants utilisés sont du carbone.
